# EUROPEAN PATENT SPECIFICATION

(11) **EP 2 030 603 B1**
(45) Date of publication and mention of the grant of the patent: **12.10.2016**
(21) Application number: 07016924.8
(22) Date of filing: 29.08.2007
(51) Int. Cl.: A61K 6/00, A61K 6/083

(54) **Dental adhesive composition**
Dentalklebstoffzusammensetzung
Composition dentaire adhésive

(43) Date of publication of application: 04.03.2009
(73) Proprietor: Dentsply DeTrey GmbH, 78467 Konstanz (DE)
(72) Inventor: Klee, Joachim E., 78315 Radolfzell (DE); Lehmann, Uwe, 78467 Konstanz (DE)
(74) Representative: Blodig, Wolfgang

(56) References cited:
- EP-A- 0 980 682
- EP-A1- 1 285 947
- EP-A1- 1 454 911
- EP-A1- 1 849 450
- DATABASE WPI Week 200538 Derwent Publications Ltd., London, GB; AN 2005-369189 XP002466052 & JP 2005 126398 A (TOKUYAMA DENTAL KK) 19 May 2005 (2005-05-19)
- SALZ U, ZIMMERMANN J, ZEUNER F, MOSZNER N.: "Hydrolytic stability of self-etching adhesive systems", J ADHES DENT, vol. 7, no. 2, 24 June 2005 (2005-06-24), pages 107-116,

## Description

### Field of the Invention

The present invention relates to a dental adhesive composition having bactericidal activity and high storage stability prior to curing and having and bacteriostatic activity when cured, while providing high mechanical resistance and high adhesion over the entire lifetime of the restoration after curing. The dental adhesive composition of the present invention may be a one-part composition having high storage stability prior to curing and high adhesive strength and long term bacteriostatic activities after curing without leaching of antimicrobial substances from the cured adhesive film. The present invention also relates to a process for the preparation of the dental adhesive composition.

### Background of the Invention

Dental adhesives containing monomers having antimicrobially active groups are known. USA 5,733,949 discloses an antimicrobial adhesive composition for dental use comprising an antimicrobial monomer, a monomer containing at least one acidic group, and a monomer containing at least one alcoholic hydroxy group, in combination with water and a polymedrization catalyst. EP-A 1 285 947 discloses an antibacterial composition comprising an antibacterial salt compound, an acid group containing polymerizable monomer, a hydrophilic polymerizable monomer, and water.

The antimicrobial groups used in the prior art may show cytotoxic effects when leaching from the adhesive layer. Since the antimicrobial adhesive compositions known from the prior art contain hydrolyzable groups, the antimicrobial groups may be hydrolyzed from the adhesive layer and may leach from the adhesive layer. A leaching of cytotoxic groups may occur due to hydrolytic cleavage of a side chain containing the antimicrobial group. The leaching of cytotoxic groups may also occur due to hydrolytic cleavage of a polymer backbone containing the antimicrobial group. Due to the leaching of the antimicrobial groups by hydrolytic cleavage, the compositions known from the prior art cannot be formulated as strongly acidic compositions. Accordingly, EP-A 1 285 947 requires a basic compound selected from alkali metal hydroxides, strong basic acid salts not having an aromatic group, and aliphatic amines for improving the storage stability of the composition by adjusting the pH in a range of from 1.5 to 4.5. Moreover, hydrolysis of the polymerizable monomers and the leaching of antimicrobial substances leads to a deterioration of the adhesive bonding to the tooth structure and a weakening of the polymeric network. Moreover, the presence of monomers such as HEMA in large proportion leads to swellable cured products which tend to swell and hydrolyze in the oral cavity. Moreover, ethylene glycol as the ultimate hydrolysis product of HEMA further accelerates the deterioration of the properties of the adhesive composition before and after curing. Accordingly, a composition known from EP-A 1 285 947, which contains about 30 percent by weight of HEMA and an acidic polymerizable (meth)acrylate ester may show a satisfactory initial adhesion to dentin whereas the adhesion deteriorates over time due to the swelling and hydrolysis of the cured adhesive.

Accordingly, it is a problem of the present invention to provide a dental adhesive composition having high storage stability and bactericidal activity prior to curing and having bacteriostatic activity when cured, while providing high mechanical resistance and adhesion over the entire lifetime of the restoration after curing. Moreover, it is a problem of the present invention to provide a dental adhesive composition from which the leaching of antimicrobial molecules may be avoided.

### Summary of the Invention

Accordingly, the present invention provides an aqueous polymerizable dental adhesive composition, which comprises
(i) 10 to 90 percent by weight based on the total weight of the composition of a mixture of hydrolysis-stable polymerizable monomers of the following formula (I):

   R(A)ₘ(B)ₙ (I)

   wherein
   - R: is a single bond or a m+n valent organic group having 1 to 20 carbon atoms and optionally 1 to 5 atoms selected from the group of nitrogen, oxygen and sulfur atoms;
   - A: independently represents a moiety of the following formula (II): 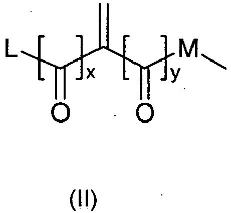
   wherein
   - L: is a hydrogen atom, a C₁₋₆ alkyl group, a C₃₋₈ cycloalkyl group, a C₆₋₁₄ aryl group or a group of the following formula (III) 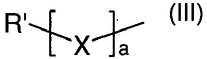 wherein
   R' is a hydrogen atom, a C₁₋₆ alkyl group, a C₃₋₈ cycloalkyl group, or a C₆₋₁₄ aryl group;
   X is O, S, NH or NR" wherein R" is a C₁₋₆ alkyl group, a C₃₋₈ cycloalkyl group, a C₆₋₁₄ aryl group;
   a is 0 or 1;
   - M: is a group of the following formula (IV) 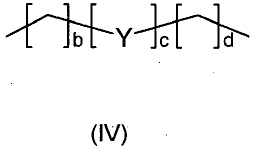 wherein
   Y is O, S, NH or NR"' wherein R'" is a C₁₋₆ alky) group, a C₃₋₈ cycloalkyl group, a C₆₋₁₄ aryl group;
   b is an integer of from 0 to 3;
   c is 0 or 1;
   d is an integer of from 0 to 3; provided that when b is 0, then Y cannot be O or S;
   x is 0 or 1;
   y is 0; provided that x and y cannot be both 1, and
   provided that x cannot be 1 when L or a group of formula (III) is a hydrogen atom, and
   provided that when x is 0 then a is also 0, and b is greater than 0 or c is 0;
   - B: independently represents a group having antimicrobial activity or an acidic group;
   - m: is an integer of from 1 to 5;
   - n: is an integer of 0 to 3; and
   - m+n: is at least 2;
(ii) a water-soluble organic solvent and
(iii) an initiator and a stabilizer; wherein the portion of antimicrobial monomers of formula (I) wherein n is greater than 0 and at least one B is a group having antimicrobial activity is at least 0.01 percent by weight based on the total weight of the mixture, and wherein the portion of acidic monomers of formula (I) wherein n is greater than 0 and at least one B is an acidic group is at least 0.1 percent by weight based on the total weight of the mixture.

Moreover, the present invention relates to a process for the preparation of a dental adhesive composition as defined by any one of claims 1 to 10, which comprises the step of mixing
(i) 10 to 90 percent by weight based on the total weight of the composition of a mixture of hydrolysis-stable polymerizable monomers of the following formula (I):

   R(A)ₘ(B)ₙ (I)

   wherein
   - R: is a single bond or a m+n valent organic group having 1 to 20 carbon atoms and optionally 1 to 5 atoms selected from the group of nitrogen, oxygen and sulfur atoms;
   - A: independently represents a moiety of the following formula (II): 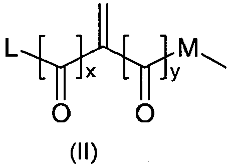 wherein

   - L: is a hydrogen atom, a C₁₋₆ alkyl group, a C₃₋₈ cycloalkyl group, a C₆₋₁₄ aryl group or a group of the following formula (III) 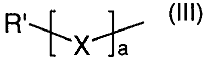 wherein
   R' is a hydrogen atom, a C₁₋₆ alkyl group, a C₃₋₈ cycloalkyl group, or a C₆₋₁₄ aryl group;
   X is O, S, NH or NR" wherein R" is a C₁₋₆ alkyl group, a C₃₋₈ cycloalkyl group, a C₆₋₁₄ aryl group;
   a is 0 or 1;
   - M: is a group of the following formula (IV) 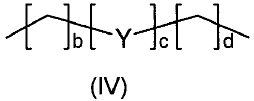 wherein
   Y is O, S, NH or NR'" wherein R'" is a C₁₋₆ alkyl group, a C₃₋₈ cycloalkyl group, a C₆₋₁₄ aryl group;
   b is an integer of from 1 to 3;
   c is 0 or 1;
   d is an integer of from 0 to 3; provided that when b is 0, then Y cannot be O or S;
   x is 0 or 1;
   y is 0 provided that x and y cannot be both 1, and provided that x cannot be 1 when L or a group of formula (III) is a hydrogen atom, and provided that when x is 0 then a is also 0, and b is greater than 0 or c is 0;
   - B: represents

   -V-W

   wherein
   V represents a single bond or a hydrolyzable linker,and
   W represents a group having antimicrobial activity;
   - m: is an integer of from 1 to 5;
   - n: is an integer of 0 to 3; and
   - m+n: is at least 2;
(ii) a water-soluble organic solvent and
(iii) an initiator system;
wherein the portion of antimicrobial monomers of formula (I) wherein n is greater than 0 and at least one B is a group having antimicrobial activity is at least 0.01 percent by weight based on the total weight of the mixture, and wherein the portion of acidic monomers of formula (I) wherein n is greater than 0 and at least one B is an acidic group is at least 0.1 percent by weight based on the total weight of the mixture.

### Detailed Description of the Preferred Embodiments

The aqueous dental adhesive composition according to the invention contains a mixture of-hydrolysis-stable polymerizable monomers. A portion of the hydrolysis-stable polymerizable monomers contains an antimicrobial group. A portion of the hydrolysis-stable polymerizable monomers contains an acidic group. A portion of the hydrolysis-stable polymerizable monomers contains a crosslinking polymerizable monomer. The polymerizable monomers are hydrolysis-stable. Specifically, the polymerizable monomers do not contain groups such as ester groups, in the main chain which hydrolyze in aqueous media at pH 3 at room temperature within one month.

The polymerizable monomers are compounds of formula (I). The monomer of formula (I), comprises a moiety R, up to five polymerizable substituents A and optionally up to three groups B which are selected from acidic groups and/or antimicrobial groups. Accordingly, m is an integer of from 1 to 5 and n is an integer of from 0 to 3. The sum of m an n is at least 2. Accordingly, in case of a single polymerizable substituents A, the polymerizable monomer comprises an acidic group or an antimicrobial group.

Moiety A is an organic group having 1 to 20 carbon atoms and optionally 1 to 5 atoms selected from the group of nitrogen, oxygen and sulfur atoms. In a preferred embodiment, R is an organic group having 1 to 6 carbon atoms. The organic group has a valency of at least two and corresponds to the total number of substituents A and B. Accordingly, R may be divalent (n=2), trivalent (n=3), tetravalent (n=4), pentavalent (n=5), or hexavalent (n=6). Preferably, R is divalent or trivalent, most preferably divalent. A may be a hydrocarbon group which may be aliphatic and/or aromatic. The hydrocarbon group may be substituted by 1 to 6 C₁₋₄ alkyl groups provided that the total amount of carbon atoms of R does not exceed 20. Specific examples of the alkyl groups are methyl, ethyl, n-propyl, i-propyl, n-butyl, i-butyl or tert.-butyl. In a preferred embodiment, the hydrocarbon group may contain 1 to 5 oxygen atoms in the hydrocarbon group in the form of aliphatic or aromatic ether bonds, keto groups, carboxylic acid groups, or hydroxyl groups. Ester groups are not preferred in moiety R in view of hydrolysis stability of the polymerizable monomer. In case of an aliphatic group, R may be a straight chain or branched chain alkylene group or a cycloalkylene group. In case of an aromatic group, R may be an arylene group or a heteroarylene group. Specifically, R may be a divalent substituted or unsubstituted C₁ to C₂₀ alkylene group, substituted or unsubstituted C₆₋₁₄ arylene group, substituted or unsubstituted C₃ to C₂₀ cycloalkylene group, substituted or unsubstituted C₇ to C₂₀ arylenealkylenearylene group. Preferably, R represents a saturated aliphatic C₂₋₂₀ hydrocarbon chain which may contain 2 to 4 oxygen atoms, and which may be substituted by 1 to 6 C₁₋₄ alkyl groups, or R may be a substituted or unsubstituted C₇ to C₂₀ arylenealkylenearylene group which may be substituted by 1 to 6 C₁₋₄ alkyl groups.

The polymerizable monomer of formula (I) contains at least one and up to five polymerizable substituents A. In case more than one A is present, each A independently represents a moiety of the following formula (II). Accordingly, in one embodiment all A are identical. In a further embodiment, the polymerizable substituents A are different from each other.

In formula (II), A contains a polymerizable double bond. A carbonyl group may be adjacent to the double bond. Accordingly, x may be 0 or 1 and y may be 0 or 1. Two carbonyl groups adjacent to the same polymerizable double bond cannot be present in a substituent A. Accordingly, x and y cannot be both 1.

Substituent A further contains moieties L and M. L is a hydrogen atom, a C₁₋₆ alkyl group, a C₃₋₈ cycloalkyl group, a C₆₋₁₄ aryl group or a group of formula (III). Specific examples of the C₁₋₆ alkyl groups are methyl, ethyl, n-propyl, i-propyl, n-butyl, i-butyl or tert.-butyl. Examples of the C₃₋₈ cycloalkyl are cyclopropyl, cyclobutyl, cyclopentyl and cyclohexyl. Specific examples of the C₆₋₁₄ aryl groups are phenyl or naphtyl. The C₁₋₆ alkyl group, the C₃₋₁₄ cycloalkyl group and the C₆₋₁₄ aryl group may optionally be substituted by one or more members of the group selected from a C₁₋₄ alkyl group, C₁₋₄ alkoxy group, and a phenyl. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

When L is a group of formula (III) then R' is a hydrogen atom, a C₁₋₆ alkyl group, a C₃₋₈ cycloalkyl group, or a C₆₋₁₄ aryl group. Specific examples of the C₁₋₆ alkyl groups are methyl, ethyl, n-propyl, i-propyl, n-butyl, i-butyl or tert.-butyl. Examples of the C₃₋₈ cycloalkyl are cyclopropyl, cyclobutyl, cyclopentyl and cyclohexyl. Specific examples of the C₆₋₁₄ aryl groups are phenyl or naphtyl. The C₁₋₆alkyl group, the C₃₋₁₄ cycloalkyl group and the C₆₋₁₄ aryl group may optionally be substituted by one or more members of the group selected from a C₁₋₄ alkyl group, C₁₋₄ alkoxy group, and a phenyl. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

When L is a group of formula (III) then X is O, S, NH or NR" wherein R" is a C₁₋₆ alkyl group, a C₃₋₈ cycloalkyl group, a C₆₋₁₄ aryl group. Specific examples of the C₁₋₆ alkyl groups are methyl, ethyl, n-propyl, i-propyl, n-butyl, i-butyl or tert.-butyl. Examples of the C₃₋₈ cycloalkyl are cyclopropyl, cyclobutyl, cyclopentyl and cyclohexyl. Specific examples of the C₆₋₁₄ aryl groups are phenyl or naphtyl. The C₁₋₆alkyl group, the C₃₋₁₄ cycloalkyl group and the C₆₋₁₄ aryl group may optionally be substituted by one or more members of the group selected from a C₁₋₄ alkyl group, C₁₋₄ alkoxy group, and a phenyl. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

When L is a group of formula (III) then a is 0 or 1.

In a group of formula (II), M is a group of formula (IV). When b, c, and d in Formula (IV) is 0 then M represents a single bond.

When M is not a single bond, then Y in formula (IV) is O, S, NH or NR'" wherein R'" is a C₁₋₆ alkyl group, a C₃₋₈ cycloalkyl group, a C₆₋₁₄ aryl group. Specific examples of the C₁₋₆ alkyl groups are methyl, ethyl, n-propyl, i-propyl, n-butyl, i-butyl or tert.-butyl. Examples of the C₃₋₈ cycloalkyl are cyclopropyl, cyclobutyl, cyclopentyl and cyclohexyl. Specific examples of the C₆₋₁₄ aryl groups are phenyl or naphtyl. The C₁₋₆alkyl group, the C₃₋₁₄ cycloalkyl group and the C₆₋₁₄ aryl group may optionally be substituted by one or more members of the group selected from a C₁₋₄ alkyl group, C₁₋₄ alkoxy group, and a phenyl. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

When M is not a single bond, then b is an integer of from 0 to 3; c is 0 or 1; d is an integer of from 0 to 3.

In formula (IV), x cannot be 1 when L or a group of formula (III) is a hydrogen atom. Still further, an hetero atom such as oxygen, sulfur and nitrogen cannot be adjacent to the polymerizable double bond. Accordingly, when x is 0 then a is also 0; and when y is 0 then b is greater than 0 or c is 0.

A compound of formula (I) may contain up to 3 substituents B which may be the same or different and independently represent a group having antimicrobial activity or an acidic group.

The groups B having antimicrobial activity are selected from 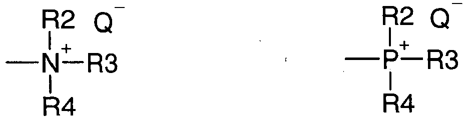 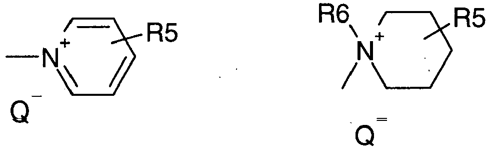 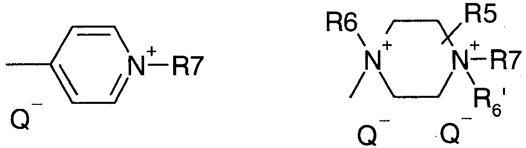 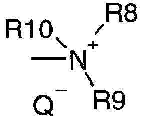 wherein R2, R3, R4 and R5 independently represent a C₁₋₁₈ alkyl group, which may be substituted by one or more substituents selected from a hydroxyl group, a halogen atom or a phenyl group optionally substituted by a C₁₋₆ alkyl group, a C₁₋₆ alkoxy group, a halogen atom, a vinyl group or a cyano group, or two substituents selected from R2, R3, and R4 together represent an C₂₋₆ alkylene group forming with the nitrogen atom or phosphorous atom to which they are bound a 3 to 7 membered ring; R6 and R6' is present or absent and when R6 or R6' are present, an anionic group Q⁻ is also present and R6 or R6' independently represent a hydrogen atom or a C₁₋₁₈ alkyl group, provided that at least one R6 and R6' is present, or R5 and R6 or R6' together represent an C₂₋₆ alkylene group, R7 represents a C₆₋₁₈ alkyl group, which may be substituted by one or more halogen atoms, or R6 or R6' and R7 together represent an C₂₋₆ alkylene group, R8 and R9 independently represent a C₆₋₁₈ alkyl group, which may be substituted by one or more substituents selected from a hydroxyl group, a halogen atom or a phenyl group optionally substituted by a C₁₋₆ alkyl group; and R10 represents a hydrogen atom or a C₁₋₁₈ alkyl group, or R10 and R8 or R9 together represent an C₂₋₆ alkylene group, Q- represents an anionic group, which is attached by a single bond or a linker to the polymerizable group. The linker may be a hydrocarbon group having up to 12 carbon atoms and may contain up to 6 heteroatoms selected from O, N and S.

Preferably, the acidic groups are selected from a sulfonic acid group, a phosphoric acid ester group, a phosphonic acid group, and a carboxylic acid group.

The mixture of the hydrolysis-stable polymerizable monomers of formula (I) is contained in the dental adhesive composition in an amount of from 10 to 90, preferably 10 to 60 percent by weight based on the total weight of the composition. More preferably, 15 to 40 percent by weight are contained.

The portion of antimicrobial monomers of formula (I) wherein n is greater than 0 and at least one B is a group having antimicrobial activity is at least 0.01 percent by weight based on the total weight of the mixture. Preferably the portion is in a range of from 0.05 to 30 percent by weight, more preferably in a range of from 0.1 to 20 percent by weight.

The portion of acidic monomers of formula (I) wherein n is greater than 0 and at least one B is an acidic group is at least 0.1 percent by weight based on the total weight of the mixture. Preferably the portion is in a range of from 0.2 to 15 percent by weight.

A further preferred group of monomers of formula (I) are those, wherein n is greater than 0, and wherein A is a moiety of formula (II) wherein L is a hydrogen atom, a C₁₋₆ alkyl group, a C₃₋₈ cycloalkyl group, or a C₆₋₁₄ aryl group, and x is 0. More preferably, A is a moiety of formula (II) wherein y is 0, L is a hydrogen atom, and M is a group of formula (IV) wherein Y is O, b and c are 1, and d is an integer of from 0 to 3.

A further preferred group of monomers of formula (I) are those, wherein A is a moiety of formula (II) wherein y is 0, L is a hydrogen atom, and M is a group of formula (IV) wherein Y is NH or NR" wherein R" is a C₁₋₆ alkyl group, a C₃₋₈ cycloalkyl group, a C₆₋₁₄ aryl group, b is 0, c is 1, and d is an integer of from 0 to 3.

The dental adhesive composition according to the present invention may contain a filler. The filler may be an inorganic or organic filler or combinations thereof. In one embodiment, a suitable inorganic particulate filler may include colloidal or submicron silicas coated with a polymer. Small amounts of pigments to allow matching of the composition to various shades of teeth can be included. In one embodiment, the filler is contained in the dental adhesive material in an amount of less than 5 % by weight.

The dental adhesive composition according to the present invention contains a polymerization initiator and a stabilizer. The initiator is not particularly limited and may be preferably a photoinitiator. Specifically, camphor quinone may be mentioned.

The dental adhesive composition according to the invention may comprise an organic water soluble solvent and/or water. The organic water soluble solvent may be selected from alcohols, such as ethanol, propanol, butanol; and/or ketones such as acetone and methyl ethyl ketone. Particularly preferred is acetone, ethanol and/or tert-butanol. Preferably, the total amount of solvent and, if present, water in the dental adhesive composition is in the range of from 10 to 10 to 90 percent by weight, preferably from 40 to 60 percent by weight.

Preferably, the molecular weight of the compound of formula (I) is in the range of from 100 to 1000, more preferably up to 500.

The dental adhesive composition according to the present invention may further contain a reactive diluent. Specific examples of a reactive diluent is selected from (meth) acrylic acid and itaconic acid.

The dental adhesive composition preferably has an adhesion to dentine of at least 10 MPa.

The dental adhesive composition according to the invention is preferably a one-part composition. A one-part composition means that the dental adhesive composition of the present invention is contained in only one container which may be stored and allows application of the dental adhesive composition without any mixing and without any special equipment before the application.

A hydrolysis-stable polymerizable monomers of formula (I) may be prepared according-to the methods as disclosed in WO02/13768, WO03/013444, WO03/035013; WO2004/078100, WO2005/063778, EP 05 022 930.1, and EP 06 021 540.7.

The invention will now be illustrated in further detail with reference to the following Examples.

### Example 1

### 1-[3.7-Dioxa-4-oxo-5-methylene-octadecyl]pyridinium bromide

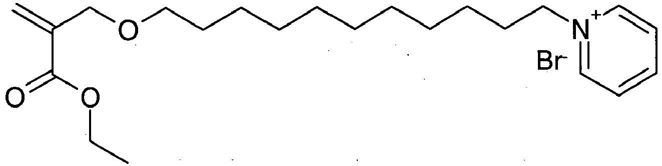

As solution of 20.0 g (0.055 mol) of ethyl 2-[11-bromo-2-oxatridecyl]acrylate and 4.78 g (0.0605 mol) pyridine dissolved in 200 ml toluene is stirred at 100 °C for 2 h. After cooling of the reaction mixture the product is isolated, washed with ether and dried in vacuum at rotation evaporator at 40 °C. Yield: 12.95 g (53 % of th.)

### Example 2

### 1-[3.7-Dioxa-4-oxo-5-methylene-octadecyl]-tri-tert.-butyl phosphonium bromide

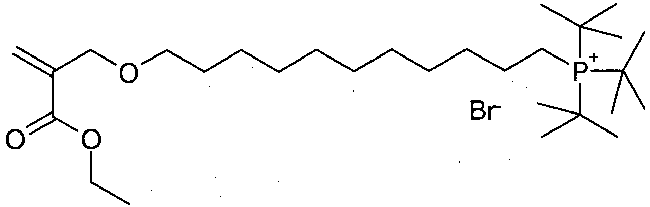

As solution of 14.67 g (0.040 mol) of ethyl 2-[11-bromo-2-oxatridecyl]acrylate and 8.986 g (0.044 mol) Tri-tert.-butyl phosphine dissolved 200 ml toluene is stirred at 100 °C for 2 h. After cooling of the reaction solution the product is isolated, washed with ether and dried in vacuum at rotation evaporator at 40 °C. Yield: 8.60 g (63.7 % of th.)

### Application Example 1

0.4611 g N,N'-Bisacrylamido-N,N'-diethyl-1,3-propane, 0.3596 g Bisacrylamido-2,4,4-(2,2,4)-trimethylhexane, 0.1320 g ethyl 2-[4-dihydrogen phosphoryl-4,2-dioxapentyl]acrylate, 0.0240 g 1-[3.7-Dioxa-4-oxo-5-methylene-octadecyl]pyridinium bromide, 0.1076g 2-acrylamido-2-methyl-propane-sulfonic acid, 0.0085g camphor quinone, 0.0213 g bis(2,4,6-trimethylbenzoyl)-phenyl phosphine oxide and 0.0099 g dimethylamino benzoic acid ethyl ester are dissolved in a solvent mixture composed of 0.5850 g ethanol and 0.3150 g water. This formulation shows antimicrobial activity against *Streptococcus mutans.*

## Claims

1. A aqueous polymerizable dental adhesive composition, which comprises
(i) 10 to 90 percent by weight based on the total weight of the composition of a mixture of hydrolysis-stable polymerizable monomers of the following formula (I):
R(A)ₘ(B)ₙ (I)
wherein
R is a m+n valent organic group having 1 to 20 carbon atoms and optionally 1 to 5 atoms selected from the group of nitrogen, oxygen and sulphur atoms;
A independently represents a moiety of the following formula (II): 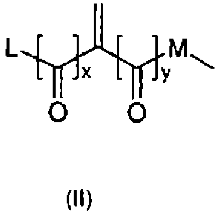 wherein
L is a hydrogen atom, a C₁₋₆ alkyl group, a C₃₋₈ cycloalkyl group, a C₆₋₁₄ aryl group or a group of the following formula (III) 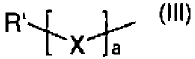 wherein
R' is a hydrogen atom, a C₁₋₆ alkyl group, a C₃₋₆ cycloalkyl group, or a C₆₋₁₄ aryl group;
X is O, S, NH or NR" wherein R" is a C₁₋₆ alkyl group, a C₃₋₈ cycloalkyl group, a C₆₋₁₄ aryl group;
a is 0 or 1;
M is a group of the following formula (IV) 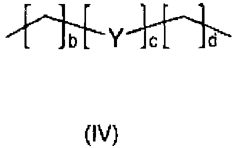 wherein
Y is O, S, NH or NR'" wherein R"' is a C₁₋₆ alkyl group, a C₃₋₈ cycloalkyl group, a C₆₋₁₄ aryl group;
b is an integer of from 0 to 3;
c is 0 or 1;
d is an integer of from 0 to 3;
provided that when b is 0, then Y cannot be O or S;
x is 0 or 1;
y is 0;
provided that x and y cannot be both 1, and
provided that x cannot be 1 when L or a group of formula (III) is a hydrogen atom, and
provided that when x is 0 then a is also 0; and
provided that b is greater than 0 or c is 0;
B independently represents a group having antimicrobial activity or an acidic
group, wherein a group B a group having antimicrobial activity contains a group selected from 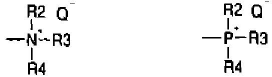 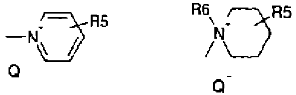 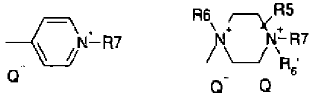 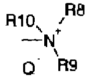 wherein
R², R³, R⁴ and R⁵ independently represent a C₁₋₁₈ alkyl group, which may be substituted by one or more substituents selected from a hydroxyl group, a halogen atom or a phenyl group optionally substituted by a C₁₋₆ alkyl group, a C₁₋₆ alkoxy group, a halogen atom, a vinyl group or a cyano group, or two substituents selected from R², R³, and R⁴ together represent an C₂₋₆ alkylene group forming with the nitrogen atom or phosphorous atom to which they are bound a 3 to 7 membered ring;
R⁶ and R^{6'} is present or absent and when R⁶ or R^{6'} are present, an anionic group Q⁻ is also present and R⁶ or R^{6'} independently represent a hydrogen atom or a C₁₋₁₈ alkyl group, provided that at least one R⁶ and R^{6'} is present,
or R⁵ and R⁶ or R^{6'} together represent an C₂₋₆ alkylene group,
R⁷ represents a C₆₋₁₈ alkyl group, which may be substituted by one or more halogen atoms, or R⁶ or R^{6'} and R⁷ together represent an C₂₋₆ alkylene group,
R⁸ and R⁹ independently represent a C₆₋₁₈ alkyl group, which may be substituted by one or more substituents selected from a hydroxyl group, a halogen atom or a phenyl group optionally substituted by a C₁₋₆ alkyl group; and
R¹⁰ represents a hydrogen atom or a C₁₋₁₈ alkyl group,
or R¹⁰ and R⁸ or R⁹ together represent an C₂₋₆ alkylene group,
Q⁻ represents an anionic group, which is attached by a single bond or a linker to the polymerizable group;
m is an integer of from 1 to 5;
n is an integer of 0 to 3; and
m+n is at least 2;
(ii) water and optionally a water-soluble organic solvent; and
(iii) an initiator and a stabilizer;
wherein the portion of antimicrobial monomers of formula (I) wherein n is greater than 0 and at least one B is a group having antimicrobial activity is at least 0.01 percent by weight based on the total weight of the mixture, and wherein the portion of acidic monomers of formula (I) wherein n is greater than 0 and at least one B is an acidic group is at least 0.1 percent by weight based on the total weight of the mixture.

2. The dental adhesive composition according to claim 1, wherein
R is a m+n valent organic group having 1 to 18 carbon atoms.

3. The dental adhesive composition according to any one of claims 1 or 2 containing monomers of formula (I), wherein n is greater than 0, and wherein A is a moiety of formula (II) wherein L is a hydrogen atom, a C₁₋₆ alkyl group, a C₃₋₈ cycloalkyl group, or a C₆₋₁₄ aryl group, and x is 0.

4. The dental adhesive composition according to claim 1, wherein A is a moiety of formula (II) wherein y is 0, L is a hydrogen atom, and M is a group of formula (IV) wherein Y is O, b and c are 1, and d is an integer of from 0 to 3.

5. The dental adhesive composition according to claim 4, wherein A is a moiety of formula (II) wherein y is 0, L is a hydrogen atom, and M is a group of formula (IV) wherein Y is NH or NR" wherein R" is a C₁₋₆ alkyl group, a C₃₋₈ cycloalkyl group, a C₆₋₁₄ aryl group, b is 0, c is 1, and d is an integer of from 0 to 3.

6. The dental adhesive composition according to any one of the preceding claims, which further contains a filler.

7. The dental adhesive composition according to any one of the preceding claims wherein the molecular weight of the compound of formula (I) is in the range of from 100 to 1000.

8. The dental adhesive composition according to any one of the preceding claims, which has an adhesion to dentine of at least 10 MPa.

9. The dental adhesive composition according to any one of the preceding claims, which is a one-part composition.

10. A process for the preparation of an aqueous dental adhesive composition as defined by any one of claims 1 to 9, which comprises the step of mixing
(i) 10 to 60 percent by weight based on the total weight of the composition of a mixture of hydrolysis-stable polymerizable monomers of the following formula (I):
R(A)ₘ(B)ₙ (I)
wherein
R is a m+n valent organic group having 1 to 20 carbon atoms and optionally 1 to 5 atoms selected from the group of nitrogen, oxygen and sulfur atoms;
A independently represents a moiety of the following formula (II): 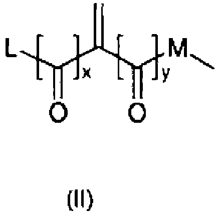 wherein
L is a hydrogen atom, a C₁₋₆ alkyl group, a C₃₋₈ cycloalkyl group, a C₆₋₁₄ aryl group or a group of the following formula (III) 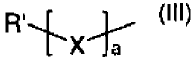 wherein
R' is a hydrogen atom, a C₁₋₆ alkyl group, a C₃₋₈ cycloalkyl group, or a C₆₋₁₄ aryl group;
X is O, S, NH or NR" wherein R" is a C₁₋₆ alkyl group, a C₃₋₈ cycloalkyl group, a C₆₋₁₄ aryl group;
a is 0 or 1;
M is a group of the following formula (IV) 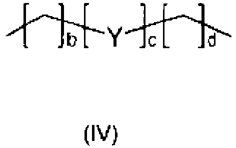 wherein
Y is O, S, NH or NR'" wherein R"' is a C₁₋₆ alkyl group, a C₃₋₈ cycloalkyl group, a C₆₋₁₄ aryl group;
b is an integer of from 1 to 3;
c is 0 or 1;
d is an integer of from 0 to 3;
provided that when b is 0, then Y cannot be O or S;
x is 0 or 1;
y is 0;
provided that x cannot be 1 when L or a group of formula (III) is a hydrogen atom, and
provided that when x is 0 then a is also 0, and
provided that b is greater than 0 or c is 0;
B independently represents a group having antimicrobial activity or an acidic group, wherein a group B having antimicrobial activity contains a group selected from 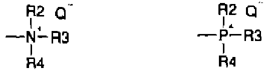 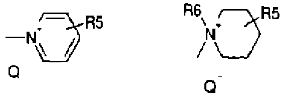 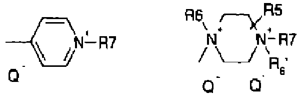 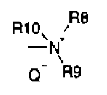 wherein
R², R³, R⁴ and R⁵ independently represent a C₁₋₁₈ alkyl group, which may be substituted by one or more substituents selected from a hydroxyl group, a halogen atom or a phenyl group optionally substituted by a C₁₋₆ alkyl group, a C₁₋₆ alkoxy group, a halogen atom, a vinyl group or a cyano group, or two substituents selected from R², R³, and R⁴ together represent an C₂₋₆ alkylene group forming with the nitrogen atom or phosphorous atom to which they are bound a 3 to 7 membered ring;
R⁶ and R^{6'} is present or absent and when R⁶ or R^{6'} are present, an anionic group Q⁻ is also present and R⁶ or R^{6'} independently represent a hydrogen atom or a C₁₋₁₈ alkyl group, provided that at least one R⁶ and R^{6'} is present,
or R⁵ and R⁶ or R^{6'} together represent an C₂₋₆ alkylene group,
R⁷ represents a C₆₋₁₈ alkyl group, which may be substituted by one or more halogen atoms, or R⁶ or R⁶ and R⁷ together represent an C₂₋₆ alkylene group,
R⁸ and R⁹ independently represent a C₆₋₁₈ alkyl group, which may be substituted by one or more substituents selected from a hydroxyl group, a halogen atom or a phenyl group optionally substituted by a C₁₋₆ alkyl group; and
R¹⁰ represents a hydrogen atom or a C₁₋₁₆ alkyl group,
or R¹⁰ and R⁸ or R⁹ together represent an C₂₋₆ alkylene group,
Q⁻ represents an anionic group, which is attached by a single bond or a linker to the polymerizable group;
m is an integer of from 1 to 5;
n is an integer of 0 to 3; and
m+n is at least 2;
(ii) water and optionally a water-soluble organic solvent; and
(iii) an initiator and a stabilizer;
wherein the portion of antimicrobial monomers of formula (I) wherein n is greater than 0 and at least one B is a group having antimicrobial activity is at least 0.01 percent by weight based on the total weight of the mixture, and wherein the portion of acidic monomers of formula (I) wherein n is greater than 0 and at least one B is an acidic group is at least 0.1 percent by weight based on the total weight of the mixture.

## Patentansprüche

1. Wässrige polymerisierbare Dentalkleber-Zusammensetzung, umfassend
(i) 10 bis 90 Gew.-%, basierend auf dem Gesamtgewicht der Zusammensetzung, einer Mischung von hydrolysestabilen polymerisierbaren Monomeren der folgenden Formel (I):
R(A)ₘ(B)ₙ (I)
worin R für eine m+n-valente organische Gruppe mit 1 bis 20 Kohlenstoffatomen steht und optional 1 bis 5 Atome, ausgewählt aus der Gruppe von Stickstoff-, Sauerstoff- und Schwefelatomen;
A unabhängig eine Gruppierung der folgenden Formel (II) darstellt 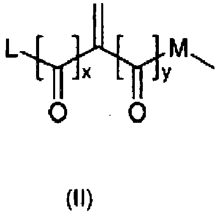 worin L für ein Wasserstoffatom, eine C₁₋₆-Alkylgruppe, eine C₃₋₈-Cycloalkylgruppe, eine C₆₋₁₄-Arylgruppe oder eine Gruppe der folgenden Formel (III) steht: 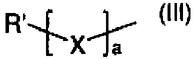
worin R' für ein Wasserstoffatom, eine C₁₋₆-Alkylgruppe, eine C₃₋₈-Cycloalkylgruppe oder eine C₆₋₁₄-Arylgruppe steht;
X für O, S, NH oder NR" steht, worin R" für eine C₁₋₆-Alkylgruppe, eine C₃₋₈-Cycloalkylgruppe, eine C₆₋₁₄-Arylgruppe steht;
a für 0 oder 1 steht;
M für eine Gruppe der folgenden Formel (IV) steht: 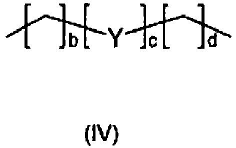
worin Y für O, S, NH oder NR'" steht, worin R'" für eine C₁₋₆-Alkylgruppe, eine C₃₋₈-Cycloalkylgruppe, eine C₆₋₁₄-Arylgruppe steht;
b für eine ganze Zahl von 0 bis 3 steht;
c für 0 oder 1 steht;
d für eine ganze Zahl von 0 bis 3 steht;
unter der Voraussetzung, dass, wenn b für 0 steht, Y nicht O oder S sein kann;
x für 0 oder 1 steht;
y für 0 steht;
unter der Voraussetzung, dass x und y nicht beide 1 sein können, und
unter der Voraussetzung, dass x nicht 1 sein kann, wenn L oder eine Gruppe der Formel (III) ein Wasserstoffatom ist, und
unter der Voraussetzung, dass, wenn x für 0 steht, a ebenfalls für 0 steht; und unter der Voraussetzung, dass b größer als 0 ist oder c 0 ist;
B unabhängig eine Gruppe mit antimikrobieller Wirkung darstellt oder eine Säuregruppe, wobei die Gruppe B mit antimikrobieller Wirkung eine Gruppe enthält, die ausgewählt ist aus: 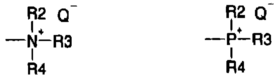 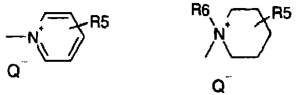 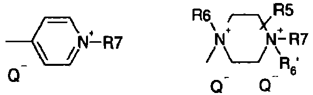 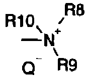
worin R², R³, R⁴ und R⁵ unabhängig eine C₁₋₁₈-Alkylgruppe darstellen, die mit einem oder mehreren Substituenten substituiert sein kann, ausgewählt aus einer Hydroxylgruppe, einem Halogenatom oder einer optional mit einer C₁₋₆-Alkylgruppe, einer C₁₋₆-Alkoxygruppe, einem Halogenatom, einer Vinylgruppe, oder einer Cyanogruppe substituierten Phenylgruppe, oder zwei Substituenten, ausgewählt aus R², R³ und R⁴, zusammen eine C₂₋₆-Alkylengruppe darstellen, die mit dem Stickstoff- oder Phosphoratom, an das sie gebunden sind, einen 3-bis 7-gliedrigen Ring bilden;
R⁶ und R^{6'} vorhanden oder nicht vorhanden sind, und wenn R⁶ oder R^{6'} vorhanden ist, ist eine ionische Gruppe Q- ebenfalls vorhanden und, unter der Voraussetzung, dass wenigstens einer von R⁶ oder R⁶ vorhanden ist, R⁶ oder R^{6'} unabhängig eine Wasserstoffatom oder eine C₁₋₁₈-Alkylgruppe darstellen,
oder R⁵ und R⁶ oder R^{6'} stellen zusammen eine C₂₋₆-Alkylengruppe dar,
R⁷ eine C₆₋₁₈-Alkylgruppe darstellt, die mit einem oder mehreren Halogenatomen substituiert sein kann oder R⁶ oder R^{6'} und R⁷ stellen zusammen eine C₂₋₆-Alkylengruppe dar,
R⁸ und R⁹ unabhängig eine C₆₋₁₈-Alkylgruppe darstellen, die mit einem oder mehreren Substituenten, ausgewählt aus einer Hydroxylgruppe, einem Halogenatom oder einer optional mit einer C₁₋₆-Alkylgruppe substituierten Phenylgruppe, substituiert sein kann; und
R¹⁰ ein Wasserstoffatom oder eine C₁₋₁₈-Alkylgruppe darstellt,
oder R¹⁰ und R⁸ oder R⁹ zusammen eine C₂₋₆-Alkylengruppe darstellen,
Q- eine anionische Gruppe darstellt, die über eine Einfachbindung oder einen Linker mit der polymerisierbaren Gruppe verbunden ist;
m für eine ganze Zahl von 1 bis 5 steht;
n für eine ganze Zahl von 0 bis 3 steht; und
m+n wenigstens 2 ist;
(ii) Wasser und optional ein wasserlösliches Lösungsmittel; und
(iii) einen Initiator und einen Stabilisator;
wobei der Anteil der antimikrobiellen Monomere in Formel (I), worin n größer als 0 ist und wenigstens ein B eine Gruppe mit antimikrobieller Wirkung mit einem Anteil von wenigstens 0,01 Gew.-% ist, basierend auf dem Gesamtgewicht der Mischung, und wobei der Anteil an Säuremonomeren in Formel (I), worin n größer als 0 ist und wenigstens ein B eine Säuregruppe mit einem Anteil von wenigstens 0,1 Gew.-% ist, basierend auf dem Gesamtgewicht der Mischung.

2. Dentalkleber-Zusammensetzung nach Anspruch 1, worin R eine m+n-valente organische Gruppe mit 1 bis 18 Kohlenstoffatomen ist.

3. Dentalkleber-Zusammensetzung nach Anspruch 1 oder 2, enthaltend Monomere der Formel (I), worin n größer als 0 ist und worin A für eine Gruppierung der Formel (II) steht, worin L für ein Wasserstoffatom, eine C₁₋₆-Alkylgruppe, eine C₃₋₈-Cycloalkylgruppe oder eine C₆₋₁₄-Arylgruppe steht, und x für 0 steht.

4. Dentalkleber-Zusammensetzung nach Anspruch 1, worin A für eine Gruppierung der Formel (II) steht, worin y für 0 steht, L für ein Wasserstoffatom steht und M für eine Gruppe der Formel (IV) steht, worin Y für O steht, b und c für 1 stehen und d für eine ganze Zahl von 0 bis 3 steht.

5. Dentalkleber-Zusammensetzung nach Anspruch 4, worin A für eine Gruppierung der Formel (II) steht, worin y für 0 steht, L für ein Wasserstoffatom steht und M für eine Gruppe der Formel (IV) steht, worin Y für NH oder NR^{"} steht, wobei R^{"} für eine C₁₋₆-Alkylgruppe, eine C₃₋₈-Cycloalkylgruppe, eine C₆₋₁₄-Arylgruppe steht, b für 0 steht, c für 1 steht und d für eine ganze Zahl von 0 bis 3 steht.

6. Dentalkleber-Zusammensetzung nach einem der vorhergehenden Ansprüche, die weiterhin einen Füllstoff enthält.

7. Dentalkleber-Zusammensetzung nach einem der vorhergehenden Ansprüche, worin das Molekulargewicht der Verbindung der Formel (I) in einem Bereich von 100 bis 1000 liegt.

8. Dentalkleber-Zusammensetzung nach einem der vorhergehenden Ansprüche, die eine Haftkraft zu Dentin von wenigstens 10 MPa aufweist.

9. Dentalkleber-Zusammensetzung nach einem der vorhergehenden Ansprüche, die eine Einkomponenten-Zusammensetzung ist.

10. Verfahren zum Herstellen einer wässrigen polymerisierbaren Dentalkleber-Zusammensetzung wie in einem der Ansprüche 1 bis 9 definiert, umfassend die Schritte des Mischens von
(i) 10 bis 90 Gew.-%, basierend auf dem Gesamtgewicht der Zusammensetzung, einer Mischung von hydrolysestabilen polymerisierbaren Monomeren der folgenden Formel (I):
R(A)ₘ(B)ₙ (I)
worin R für eine m+n-valente organische Gruppe mit 1 bis 20 Kohlenstoffatomen steht und optional 1 bis 5 Atome, ausgewählt aus der Gruppe von Stickstoff-, Sauerstoff- und Schwefelatomen;
A unabhängig eine Gruppierung der folgenden Formel (II) darstellt 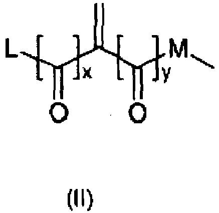 worin L für ein Wasserstoffatom, eine C₁₋₆-Alkylgruppe, eine C₃₋₈-Cycloalkylgruppe, eine C₆₋₁₄-Arylgruppe oder eine Gruppe der folgenden Formel (III) steht: 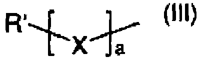
worin R' für ein Wasserstoffatom, eine C₁₋₆-Alkylgruppe, eine C₃₋₈-Cycloalkylgruppe oder eine C₆₋₁₄-Arylgruppe steht;
X für O, S, NH oder NR" steht, worin R" für eine C₁₋₆-Alkylgruppe, eine C₃₋₈-Cycloalkylgruppe, eine C₆₋₁₄-Arylgruppe steht;
a für 0 oder 1 steht;
M für eine Gruppe der folgenden Formel (IV) steht: 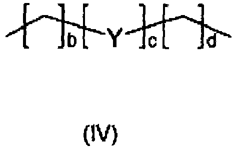
worin Y für O, S, NH oder NR'" steht, worin R'" für eine C₁₋₆-Alkylgruppe, eine C₃₋₈-Cycloalkylgruppe, eine C₆₋₁₄-Arylgruppe steht;
b für eine ganze Zahl von 1 bis 3 steht;
c für 0 oder 1 steht;
d für eine ganze Zahl von 0 bis 3 steht;
unter der Voraussetzung, dass, wenn b 0 ist, Y nicht O oder S sein kann;
x für 0 oder 1 steht;
y für 0 steht;
unter der Voraussetzung, dass x nicht 1 sein kann, wenn L oder eine Gruppe der Formel (III) ein Wasserstoffatom ist, und
unter der Voraussetzung, dass, wenn x für 0 steht, a ebenfalls für 0 steht; und unter der Voraussetzung, dass b größer als 0 ist oder c 0 ist;
B unabhängig eine Gruppe mit antimikrobieller Wirkung darstellt oder eine Säuregruppe, wobei die Gruppe B mit antimikrobieller Wirkung eine Gruppe enthält, die ausgewählt ist aus: 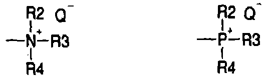 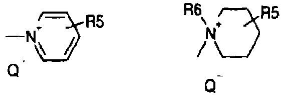 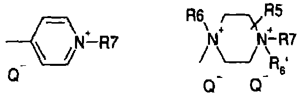 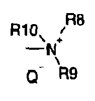
worin R², R³, R⁴ und R⁵ unabhängig eine C₁₋₁₈-Alkylgruppe darstellen, die mit einem oder mehreren Substituenten substituiert sein kann, ausgewählt aus einer Hydroxylgruppe, einem Halogenatom oder einer optional mit einer C₁₋₆-Alkylgruppe, einer C₁₋₆-Alkoxygruppe, einem Halogenatom, einer Vinylgruppe, oder einer Cyanogruppe substituierten Phenylgruppe, oder zwei Substituenten ausgewählt aus R², R³ und R⁴ zusammen eine C₂₋₆-Alkylengruppe darstellen, die mit dem Stickstoff- oder Phosphoratom, an das sie gebunden sind, einen 3- bis 7-gliedrigen Ring bilden;
R⁶ und R^{6'} vorhanden oder nicht vorhanden sind, und wenn R⁶ oder R^{6'} vorhanden ist, ist eine ionische Gruppe Q⁻ ebenfalls vorhanden und, unter der Voraussetzung, dass wenigstens einer von R⁶ oder R^{6'} vorhanden ist, R⁶ oder R^{6'} unabhängig eine Wasserstoffatom oder eine C₁₋₁₈-Alkylgruppe darstellen,
oder R⁵ und R⁶ oder R^{6'} stellen zusammen eine C₂₋₆-Alkylengruppe dar,
R⁷ eine C₆₋₁₈-Alkylgruppe darstellt, die mit einem oder mehreren Halogenatomen substituiert sein kann oder R⁶ oder R^{6'} und R⁷ stellen zusammen eine C₂₋₆-Alkylengruppe dar,
R⁸ und R⁹ unabhängig eine C₆₋₁₈-Alkylgruppe darstellen, die mit einem oder mehreren Substituenten, ausgewählt aus einer Hydroxylgruppe, einem Halogenatom oder einer optional mit einer C₁₋₆-Alkylgruppe substituierten Phenylgruppe, substituiert sein kann; und
R¹⁰ ein Wasserstoffatom oder eine C₁₋₁₈-Alkylgruppe darstellt,
oder R¹⁰ und R⁸ oder R⁹ zusammen eine C₂₋₆-Alkylengruppe darstellen,
Q- eine anionische Gruppe darstellt, die über eine Einfachbindung oder einen Linker mit der polymerisierbaren Gruppe verbunden ist;
m für eine ganze Zahl von 1 bis 5 steht;
n für eine ganze Zahl von 0 bis 3 steht; und
m+n wenigstens 2 ist;
(ii) Wasser und optional ein wasserlösliches Lösungsmittel; und
(iii) einen Initiator und einen Stabilisator;
wobei der Anteil der antimikrobiellen Monomere in Formel (I), worin n größer als 0 ist und wenigstens ein B eine Gruppe mit antimikrobieller Wirkung mit einem Anteil von wenigstens 0,01 Gew.-% ist, basierend auf dem Gesamtgewicht der Mischung, und wobei der Anteil an Säuremonomeren in Formel (I), worin n größer als 0 ist und wenigstens ein B eine Säuregruppe mit einem Anteil von wenigstens 0,1 Gew.-% ist, basierend auf dem Gesamtgewicht der Mischung.

## Revendications

1. Composition aqueuse d'adhésif dentaire polymérisable, qui comprend :
(i) 10 à 90 % en poids, sur la base du poids total de la composition, d'un mélange de monomères polymérisables stables à l'hydrolyse, répondant à la formule (I) suivante:
R(A)ₘ(B)ₙ (I)
dans laquelle :
R représente un groupe organique de valence m+n ayant 1 à 20 atomes de carbone et, facultativement, 1 à 5 atomes choisis dans le groupe des atomes d'azote, d'oxygène et de soufre ;
A représente indépendamment un groupement répondant à la formule (II) suivante : 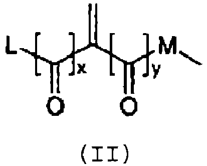 dans laquelle :
L représente un atome d'hydrogène, un groupe alkyle en C₁ à C₆, un groupe cycloalkyle en C₃ à C₈, un groupe aryle en C₆ à C₁₄ ou un groupe répondant à la formule (III) suivante : 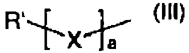 dans laquelle :
R' représente un atome d'hydrogène, un groupe alkyle en C₁ à C₆, un groupe cycloalkyle en C₃ à C₈ ou un groupe aryle en C₆ à C₁₄ ;
X représente O, S, un groupe NH ou NR", dans lequel R'' représente un groupe alkyle en C₁ à C₆, un groupe cycloalkyle en C₃ à C₈ ou un groupe aryle en C₆ à C₁₄ ; a est égal à 0 ou 1 ;
M représente un groupe répondant à la formule (IV) suivante 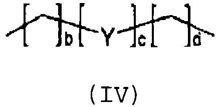 dans laquelle :
Y représente O, S, un groupe NH ou Nu"', dans lequel R''' représente un groupe alkyle en C₁ à C₆, un groupe cycloalkyle en C₃ à C₈ ou un groupe aryle en C₆ à C₁₄ ;
b représente un nombre entier de 0 à 3 ;
c est égal à 0 ou 1 ;
d représente un nombre entier de 0 à 3 ;
sous réserve que, lorsque b est égal à 0, alors Y ne peut représenter O ou S ;
x est égal à 0 ou 1 ;
y est égal à 0 ;
sous réserve que x et y ne puissent être tous deux égaux à 1, et
sous réserve que x ne puisse être égal à 1 lorsque L ou un groupe de formule (III) représente un atome d'hydrogène, et
sous réserve que, lorsque x est égal à 0, alors a est également égal à 0 ; et
sous réserve que b est supérieur à 0 ou c est égal à 0 ;
B représente indépendamment un groupe ayant une activité antimicrobienne ou un groupe acide, où un groupe B consistant en un groupe ayant une activité antimicrobienne contient un groupe choisi entre des groupes 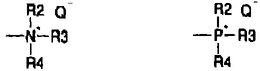 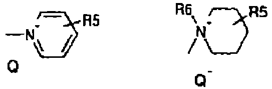 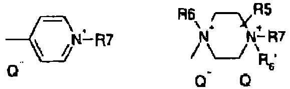 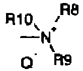 dans lesquels :
R², R³, R⁴ et R⁵ représentent indépendamment un groupe alkyle en C₁ à C₁₈, qui peut être substitué avec un ou plusieurs substituants choisis entre un groupe hydroxyle, un atome d'halogène et un groupe phényle facultativement substitué avec un groupe alkyle en C₁ à C₆, un groupe alkoxy en C₁ à C₆, un atome d'halogène, un groupe vinyle ou un groupe cyano, ou bien deux substituants choisis entre R², R³ et R⁴ représentent conjointement un groupe alkylène en C₂ à C₆ formant avec l'atome d'azote ou l'atome de phosphore auquel ils sont liés un noyau tri- à heptagonal ;
R⁶ et R^{6'} est présent ou absent et, lorsque R⁶ ou R^{6'} est présent, un groupe anionique Q⁻ est également présent et R⁶ ou R^{6'} représente indépendamment un atome d'hydrogène ou un groupe alkyle en C₁ à C₁₈, sous réserve qu'au moins un de R⁶ et R^{6'} soit présent,
ou R⁵ et R⁶ ou R^{6'} représentent conjointement un groupe alkylène en C₂ à C₆,
R⁷ représente un groupe alkyle en C₆ à C₁₈, qui peut être substitué avec un ou plusieurs atomes d'halogène, ou bien R⁶ ou R^{6'} et R⁷ représentent conjointement un groupe alkylène en C₂ à C₆,
R⁸ et R⁹ représentent indépendamment un groupe alkyle en C₆ à C₁₈, qui peut être substitué avec un ou plusieurs substituants choisis entre un groupe hydroxyle, un atome d'halogène et un groupe phényle facultativement substitué avec un groupe alkyle en C₁ à C₆ ; et
R¹⁰ représente un atome d'hydrogène ou un groupe alkyle en C₁ à C₁₈,
ou R¹⁰ et R⁸ ou R⁹ représentent conjointement un groupe alkylène en C₂ à C₆,
Q⁻ représente un groupe anionique, qui est fixé par une liaison simple ou un groupe de liaison au groupe polymérisable ;
m représente un nombre entier de 1 à 5 ;
n représente un nombre entier de 0 à 3 ; et
m+n est égal à au moins 2 ;
(ii) de l'eau et, facultativement, un solvant organique hydrosoluble ; et
(iii) un initiateur et un stabilisant ;
dans laquelle la portion de monomères antimicrobiens de formule (I) dans laquelle n est supérieur à 0 et au moins un groupe B est un groupe ayant une activité antimicrobienne, est égale à au moins 0,01 % en poids sur la base du poids total du mélange, et dans laquelle la portion de monomères acides de formule (I) dans laquelle n est supérieur à 0 et au moins un groupe B est un groupe acide, est égale à au moins 0,1 % en poids sur la base du poids total du mélange.

2. Composition d'adhésif dentaire suivant la revendication 1, dans laquelle :
R représente un groupe organique de valence m+n ayant 1 à 18 atomes de carbone.

3. Composition d'adhésif dentaire suivant l'une quelconque des revendications 1 et 2, contenant des monomères de formule (I), dans laquelle n est supérieur à 0, et dans laquelle A représente un groupement de formule (II) dans laquelle L représente un atome d'hydrogène, un groupe alkyle en C₁ à C₆, un groupe cycloalkyle en C₃ à C₈ ou un groupe aryle en C₆ à C₁₄, et x est égal à 0.

4. Composition d'adhésif dentaire suivant la revendication 1, dans laquelle A représente un groupement de formule (II) dans laquelle y est égal à 0, L représente un atome d'hydrogène et M représente un groupe de formule (IV) dans laquelle Y représente O, b et c sont égaux à 1 et d représente un nombre entier de 0 à 3.

5. Composition d'adhésif dentaire suivant la revendication 4, dans laquelle A représente un groupement de formule (II) dans laquelle y est égal à 0, L représente un atome d'hydrogène et M représente un groupe de formule (IV) dans laquelle Y représente un groupe NH ou NR'', dans lequel R'' représente un groupe alkyle en C₁ à C₆, un groupe cycloalkyle en C₃ à C₈, un groupe aryle en C₆ à C₁₄, b est égal à 0, c est égal à 1 et d représente un nombre entier de 0 à 3.

6. Composition d'adhésif dentaire suivant l'une quelconque des revendications précédentes, qui contient en outre une charge.

7. Composition d'adhésif dentaire suivant l'une quelconque des revendications précédentes, dans laquelle le poids moléculaire du composé de formule (I) est compris dans l'intervalle de 100 à 1000.

8. Composition d'adhésif dentaire suivant l'une quelconque des revendications précédentes, qui a une adhérence à la dentine d'au moins 10 MPa.

9. Composition d'adhésif dentaire suivant l'une quelconque des revendications précédentes, qui est une composition en une partie.

10. Procédé pour la préparation d'une composition aqueuse d'adhésif dentaire telle que définie par l'une quelconque des revendications 1 à 9, qui comprend l'étape de mélange :
(i) de 10 à 60 % en poids, sur la base du poids total de la composition, d'un mélange de monomères polymérisables stables à l'hydrolyse, répondant à la formule (I) suivante :
R(A)ₘ(B)ₙ (I)
dans laquelle :
R représente un groupe organique de valence m+n ayant 1 à 20 atomes de carbone et, facultativement, 1 à 5 atomes choisis dans le groupe des atomes d'azote, d'oxygène et de soufre ;
A représente indépendamment un groupement répondant à la formule (II) suivante : 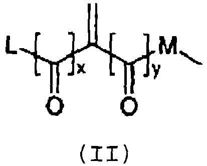 dans laquelle :
L représente un atome d'hydrogène, un groupe alkyle en C₁ à C₆, un groupe cycloalkyle en C₃ à C₈, un groupe aryle en C₆ à C₁₄ ou un groupe répondant à la formule (III) suivante : 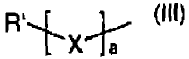 dans laquelle :
R' représente un atome d'hydrogène, un groupe alkyle en C₁ à C₆, un groupe cycloalkyle en C₃ à C₈ ou un groupe aryle en C₆ à C₁₄ ;
X représente O, S, un groupe NH ou NR'', dans lequel R'' représente un groupe
a alkyle en C₁ à C₆, un groupe cycloalkyle en C₃ à C₈ ou un groupe aryle en C₆ à C₁₄ ; est égal à 0 ou 1 ;
M représente un groupe répondant à la formule (IV) suivante 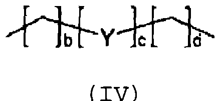 dans laquelle :
Y représente O, S, un groupe NH ou NR''', dans lequel R'" représente un groupe alkyle en C₁ à C₆, un groupe cycloalkyle en C₃ à C₈ ou un groupe aryle en C₆ à C₁₄ ;
b représente un nombre entier de 1 à 3 ;
c est égal à 0 ou 1 ;
d représente un nombre entier de 0 à 3 ;
sous réserve que, lorsque b est égal à 0, alors Y ne peut représenter O ou S ;
x est égal à 0 ou 1 ;
y est égal à 0 ;
sous réserve que x ne puisse être égal à 1 lorsque L ou un groupe de formule (III) représente un atome d'hydrogène, et
sous réserve que, lorsque x est égal à 0, alors a est également égal à 0 ; et
sous réserve que b est supérieur à 0 ou c est égal à 0 ;
B représente indépendamment un groupe ayant une activité antimicrobienne ou un groupe acide, un groupe B ayant une activité antimicrobienne contenant un groupe choisi entre des groupes 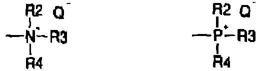 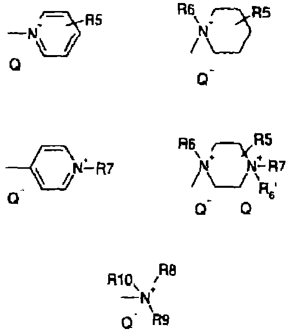 dans lesquels
+R², R³, R⁴ et R⁵ représentent indépendamment un groupe alkyle en C₁ à C₁₈, qui peut être substitué avec un ou plusieurs substituants choisis entre un groupe hydroxyle, un atome d'halogène et un groupe phényle facultativement substitué avec un groupe alkyle en C₁ à C₆, un groupe alkoxy en C₁ à C₆, un atome d'halogène, un groupe vinyle ou un groupe cyano, ou bien deux substituants choisis entre R², R³ et R⁴ représentent conjointement un groupe alkylène en C₂ à C₆ formant avec l'atome d'azote ou l'atome de phosphore auquel ils sont liés un noyau tri- à heptagonal ;
R⁶ et R^{6'} est présent ou absent et, lorsque R⁶ ou R^{6'} est présent, un groupe anionique Q⁻ est également présent et R⁶ ou R^{6'} représente indépendamment un atome d'hydrogène ou un groupe alkyle en C₁ à C₁₈, sous réserve qu'au moins un de R⁶ et R^{6'} soit présent,
ou R⁵ et R⁶ ou R^{6'} représentent conjointement un groupe alkylène en C₂ à C₆,
R⁷ représente un groupe alkyle en C₆ à C₁₈, qui peut être substitué avec un ou plusieurs atomes d'halogène, ou bien R⁶ ou R^{6'} et R⁷ représentent conjointement un groupe alkylène en C₂ à C₆,
R⁸ et R⁹ représentent indépendamment un groupe alkyle en C₆ à C₁₈, qui peut être substitué avec un ou plusieurs substituants choisis entre un groupe hydroxyle, un atome d'halogène et un groupe phényle facultativement substitué avec un groupe alkyle en C₁ à C₆ ; et
R¹⁰ représente un atome d'hydrogène ou un groupe alkyle en C₁ à C₁₈,
ou R¹⁰ et R⁸ ou R⁹ représentent conjointement un groupe alkylène en C₂ à C₆,
Q⁻ représente un groupe anionique, qui est fixé par une liaison simple ou un groupe de liaison au groupe polymérisable ;
m représente un nombre entier de 1 à 5 ;
n représente un nombre entier de 0 à 3 ; et
m+n est égal à au moins 2 ;
(ii) d'eau et, facultativement, d'un solvant organique hydrosoluble ; et
(iii) d'un initiateur et d'un stabilisant ;
dans lequel la portion de monomères antimicrobiens de formule (I) dans laquelle n est supérieur à 0 et au moins un groupe B est un groupe ayant une activité antimicrobienne, est égale à au moins 0,01 % en poids sur la base du poids total du mélange, et dans lequel la portion de monomères acides de formule (I) dans laquelle n est supérieur à 0 et au moins un groupe B est un groupe acide, est égale à au moins 0,1 % en poids sur la base du poids total du mélange.
